# EUROPÄISCHE PATENTSCHRIFT

(11) **EP 1 897 520 B1**
(45) Veröffentlichungstag und Bekanntmachung des Hinweises auf die Patenterteilung: **10.11.2010**
(21) Anmeldenummer: 06405385.3
(22) Anmeldetag: 07.09.2006
(51) Int. Cl.: A61F 9/01

(54) **Opthalmologische Vorrichtung für die refraktive Korrektur eines Auges.**
Ophthalmological device for the refractive correction of an eye.
Dispositif ophtalmologique pour la correction réfractive de l'oeil.

(43) Veröffentlichungstag der Anmeldung: 12.03.2008
(60) Teilanmeldung: 10009126.3
(73) Patentinhaber: Ziemer Holding AG, 2562 Port (CH)
(72) Erfinder: Rathjen, Christian, 28197 Bremen (DE)
(74) Vertreter: Vogel, Dany

(56) Entgegenhaltungen:
- EP-A1- 1 486 185
- US-A1- 2004 199 149
- US-A1- 2004 243 111
- US-A1- 2004 243 112
- US-B1- 6 325 792

## Beschreibung

### Technisches Gebiet

Die vorliegende Erfindung betrifft eine ophthalmologische Vorrichtung für die refraktive Korrektur eines Auges. Die Erfindung betrifft insbesondere eine ophthalmologische Vorrichtung für die refraktive Korrektur eines Auges mittels Projektion von Laserpulsen auf einen Brennpunkt im Innern des Auges für eine Auflösung von Augengewebe.

### Stand der Technik

Fehlsichtigkeiten wie Myopie (Kurzsichtigkeit), Hyperopie (Weitsichtigkeit oder Übersichtigkeit) oder Astigmatismus (Stabsichtigkeit) können heute durch refraktiv-chirurgische Behandlung dauerhaft korrigiert werden. Refraktiv-chirurgische Behandlungen sind chirurgische Eingriffe am Auge, die die optische Brechkraft des Auges ändern mit dem Ziel, diese einem gewünschten Wert möglichst gut anzunähern. Mittels Femtolasersystemen, die Pulsbreiten von typisch 10fs bis 1000fs (1fs=10⁻¹⁵s) aufweisen, können transparente Materialen im Fokus durch nichtlineare Absorption und anschliessende Wechselwirkung (z.B. Photodisruption) bearbeitet werden. Insbesondere werden in der Praxis in der Augenhomhaut (Cornea) durch Gewebeauflösung mittels Femtolaserpulsen operativ Schnitte erzeugt.

In der Patentschrift US 5,993,438 wird ein Verfahren für die refraktive Keratektomie in der Augenhomhaut mittels gepulster Laserstrahlen beschrieben. Gemäss US 5,993,438 wird durch Gewebeauflösung im Innern der Augenhornhaut ein domförmiger Hohlraum um die optische Achse erzeugt, welcher beim Kollabieren die Hornhautkrümmung geeignet verändert. Der zusammenhängende Hohlraum wird durch mehrere direkt übereinander liegende Abtragungsschichten gebildet, die zentralsymmetrisch um die optische Achse des Auges angeordnet sind. Jede der Abtragungsschichten wird durch beispielsweise spiralförmig hintereinander gereihte Laserpulse zentralsymmetrisch um die optische Achse herum erzeugt. Mit abnehmender Distanz zu der Hornhautoberfläche weisen die Abtragungsschichten jeweils einen abnehmenden Durchmesser auf. Die Distanz zwischen den Fokusdurchmessem von hintereinander folgenden Laserpulsen beträgt nach US 5,993,438 vorzugsweise das Ein- bis Zweifache des Radius einer durch einen der Laserpulse im Brennpunkt erzeugten Blase. In einer Abtragungsschicht ist das Augengewebe jeweils über eine Dicke von ungefähr 10µm aufgelöst. Die übereinander liegenden Abtragungsschichten werden jeweils direkt aneinandergrenzend erzeugt, so dass der domförmige Hohlraum zusammenhängend, ohne verbleibende Gewebebrücken gebildet wird. Das Verfahren nach US 5,993,438 eignet sich zwar für die Behandlung von Myopie, der domförmige Hohlraum ist jedoch nicht für die Korrektur von Hyperopie, Astigmatismus oder Aberrationen (Abbildungsfehler) höherer Ordnung geeignet.

Die Patentschrift US 6,325,792 beschreibt eine Vorrichtung für die Augenchirurgie mittels Femtosekundenlaser, bei welcher der Fokuspunkt in zwei Dimensionen an eine beliebige Stelle positioniert werden kann. Im Augengewebe können mittels eines Scanners unterschiedlichste Schnittformen erzeugt werden, insbesondere können auf der Hornhaut mehrere voneinander getrennte radiale Schnitte angebracht werden.

Die Patentanmeldung US 2004/0199149 beschreibt eine Vorrichtung für die laserbasierte Augenchirurgie, welche Linsenkorrekturen auf der Basis von einer Vielzahl von nebeneinander und übereinander angeordneten Mikrosphären erzeugt, wobei die Mikrosphären jeweils durch einen Puls "punktförmig" an einem Fokuspunkt gebildet werden. US 2004/0199149 beschreibt insbesondere die Anhäufung von mehreren getrennten punktförmigen Mikrosphären zu einem Cluster mit übergeordneter Ring- oder Scheibenstruktur.

### Darstellung der Erfindung

Es ist eine Aufgabe der vorliegenden Erfindung, eine neue ophthalmologische Vorrichtung für die refraktive Korrektur eines Auges mittels Laserpulsen vorzuschlagen, welche insbesondere nicht auf die Korrektur der Kurzsichtigkeit beschränkt sind.

Gemäss der vorliegenden Erfindung werden diese Ziele insbesondere durch die Elemente der unabhängigen Ansprüche erreicht. Weitere vorteilhafte Ausführungsformen gehen ausserdem aus den abhängigen Ansprüchen und der Beschreibung hervor.

Die oben genannten Ziele werden durch die vorliegende Erfindung insbesondere dadurch erreicht, dass die ophthalmologische Vorrichtung, welche einen Lichtprojektor umfasst zur Projektion von Laserpulsen auf einen Brennpunkt im Innern des Auges zur Auflösung von Augengewebe, zudem ein Positionierungsmodul und ein Abtastmodul umfasst. Das Positionierungsmodul ist eingerichtet, den Brennpunkt an unterschiedliche Ausgangspunkte zu positionieren. Das Abtastmodul ist eingerichtet, den Brennpunkt ausgehend von jeweils einem der Ausgangspunkte gemäss einem Abtastmuster für ein Bearbeitungsteilgebiet zu bewegen, wobei das Abtastmuster und die Ausgangspunkte so definiert sind, dass das Augengewebe in mehreren durch Gewebebrücken voneinander getrennten Bearbeitungsteilgebieten aufgelöst wird. Das Abtastmuster definiert beispielsweise ein Bearbeitungsteilgebiet mit einer rechteckigen, runden, elliptischen, sternförmigen oder spiralförmigen Form oder in einer Form ähnlich einer Lissajou-Figur. Zum Ablenken der Laserpulse umfasst das Abtastmodul beispielsweise einen Galvanoscanner, einen resonanten Spiegelscanner, einen akustischen optischen Modulator, einen Polygonscanner und/oder einen mikroelektromechanischen Scanner. Das Positionierungsmodul umfasst Bewegungstreiber zum mechanischen Verschieben von mindestens Teilen des Lichtprojektors und/oder einen Galvanoscanner zum Ablenken der Laserpulse. Der Lichtprojektor weist vorzugsweise eine numerische Apertur von über 0.3 auf. Die ophthalmologische Vorrichtung umfasst beispielsweise ein Steuermodul, welches eingerichtet ist, das Positionierungsmodul und das Abtastmodul so zu steuern, dass das Positionierungsmodul den Brennpunkt so an unterschiedliche Ausgangspunkte positioniert und dass das Abtastmodul den Brennpunkt ausgehend von jeweils einem der Ausgangspunkte gemäss dem Abtastmuster so bewegt, dass das Augengewebe in mehreren durch Gewebebrücken voneinander getrennten Bearbeitungsteilgebieten aufgelöst wird. Durch die Bildung einer Vielzahl von getrennten, nicht zusammenhängenden Bearbeitungsteilgebieten mit aufgelöstem Augengewebe ist es möglich die Krümmung der Augenhornhaut nicht bloss wie im Stand der Technik zentralsymmetrisch zur Korrektur einer Myopie abzuflachen, sondern für eine refraktive Korrektur die Krümmung der Augenhornhaut an beinahe beliebigen Stellen und insbesondere auch asymmetrisch zu ändern. Beispielsweise können, durch geeignete Wahl der Ausgangspunkte, mehrere der Bearbeitungsteilgebiete in einem ringförmigen Cluster im (intrastromalen) Homhautgewebe so angeordnet werden, dass eine Hyperopie korrigiert werden kann. Durch unterschiedliche Verteilung der Bearbeitungsteilgebiete in der Hornhaut, sowohl in der Tiefe als auch in der Distanz zur optischen Achse des Auges, und/oder durch mehrschichtige Anordnung der Bearbeitungsteilgebiete im Homhautgewebe kann die Homhautkrümmung zur Korrektur von Astigmatismus und Aberrationen höherer Ordnung geeignet verändert werden. Neben der Behandlung und Korrektur der Augenhornhaut ist es zudem auch möglich, durch die ophthalmologische Vorrichtung auf die gleiche Art das Gewebe der Augenlinse zu behandeln, insbesondere zur Verbesserung der Elastizität der Linse bei Altersweitsichtigkeit.

In einer bevorzugten Ausführungsvariante ist das Positionierungsmodul eingerichtet, den Brennpunkt jeweils an Ausgangspunkte auf einer ersten Bearbeitungsfläche zu positionieren, und das Abtastmodul ist eingerichtet, den Brennpunkt in dieser ersten Bearbeitungsfläche zu bewegen. Die ophthalmologische Vorrichtung umfasst zudem ein Tiefenpositionierungsmodul zum Verschieben des Brennpunkts entlang einer Projektionsachse des Lichtprojektors in eine zur ersten Bearbeitungsfläche äquidistante, z.B. parallele, zweite Bearbeitungsfläche, so dass der Brennpunkt in der zweiten Bearbeitungsfläche an unterschiedliche Ausgangspunkte positionierbar und ausgehend von jeweils einem der Ausgangspunkte gemäss dem Abtastmuster bewegbar ist. Durch die Tiefenpositionierung des Brennpunkts können mehrere Fokalflächen, z.B. Fokalebenen, eingestellt werden, welche jeweils eine Bearbeitungsfläche, z.B. Bearbeitungsebene, bilden, auf der die Ausgangspunkte jeweils definiert und das Augengewebe in Bearbeitungsteilgebieten aufgelöst wird. Die Tiefenpositionierung des Brennpunkts ermöglicht somit eine mehrschichtige Bearbeitung des Augengewebes mit jeweils einer Vielzahl von getrennten, nicht zusammenhängenden Bearbeitungsteilgebieten, in denen das Augengewebe aufgelöst wird. Vorzugsweise wird dabei die Distanz zwischen einzelnen Fokalflächen respektive Bearbeitungsflächen so bestimmt, dass bei übereinander liegenden Bearbeitungsteilgebieten benachbarter Bearbeitungsflächen, jeweils eine Gewebebrücke bestehen bleibt. Zu diesem Zweck ist das Steuermodul zudem vorzugsweise eingerichtet, das Tiefenpositionierungsmodul so zu steuern, dass beim Verschieben des Brennpunkts eine Mindestdistanz zwischen den Bearbeitungsflächen eingehalten wird, wobei die Mindestdistanz so definiert ist, dass in äquidistanten (parallelen) Bearbeitungsflächen über einander liegende Bearbeitungsteilgebiete durch Gewebebrücken voneinander getrennt sind. Der Zweck und Vorteil der Gewebebrücken besteht darin, dass definierte Abtragschichtdicken erzeugt werden können. Es hat sich nämlich herausgestellt, dass bei der Laserbearbeitung entstehende innere Gasdrucke eine Deformation des Gewebes erzeugen, die die Präzision beim schichtweisen Abtragen von grossen zusammenhängenden Schichten, wie es im Stand der Technik beschrieben wird, stark beeinträchtigt.

In einer Ausführungsvariante ist das Steuermodul eingerichtet, entsprechend einer gewünschten refraktiven Korrektur des Auges, die Anzahl der Bearbeitungsteilgebiete sowie die Ausgangspunkte für die örtliche Verteilung der Bearbeitungsteilgebiete in mehreren Bearbeitungsflächen im Innern des Auges zu bestimmen. Das Steuermodul bestimmt die örtliche Verteilung der Bearbeitungsteilgebiete beispielsweise auf Grund eines Modells des zu behandelnden Augengewebes, z.B. ein Hornhautmodel, bei vorgegebener Grösse und Form der Bearbeitungsteilgebiete und bei vorgegebenen vertikalen und horizontalen Mindestabständen einzelner Bearbeitungsteilgebiete.

In einer Ausführungsvariante ist das Steuermodul zudem eingerichtet, unterschiedliche Abtastmuster für unterschiedlich grosse Bearbeitungsteilgebiete zu wählen.

In einer bevorzugten Ausführungsvariante umfasst die Vorrichtung einen Wellenfrontdetektor zum Bestimmen eines Wellenfrontverlaufs eines durch das Auge reflektierten Lichtbündels. Das Steuermodul ist zudem eingerichtet, die Ausgangspunkte basierend auf dem bestimmten Wellenfrontverlauf festzulegen. Das heisst, das Steuermodul ist eingerichtet, die örtliche Verteilung der Bearbeitungsteilgebiete basierend auf dem bestimmten Wellenfrontverlauf festzulegen. Dadurch kann die erreichte refraktive Korrektur während der Behandlung gemessen werden und, darauf basierend, die Positionierung weiterer Bearbeitungstellgebiete, so weit nötig, bestimmt werden.

Vorzugsweise ist das Abtastmodul eingerichtet, nacheinander folgende Laserpulse so zu positionieren, dass sich ihre Fokusdurchmesser teilweise überlappen. Vorzugsweise überlappen sich ihre Fokusdurchmesser mindestens bis zur Hälfte ihres Durchmessers. Durch die Überlappung der Fokusdurchmesser können Laserpulse mit geringere Pulsenergie für die Gewebeauflösung eingesetzt werden, wodurch nur geringe mechanische Spannungen durch Gas und Kavitationsblasen im Restgewebe induziert werden, was bei der Bildung von gleichmässig dünnen Abtragsbereichen hilft und einer definierten Kollabierung der Bearbeitungsteilgebiete förderlich ist. Insbesondere zusammen mit der Verwendung hoher numerischer Aperturen, beispielsweise >0.3, insbesondere >0.4, und der damit verbundenen geringeren erforderlichen Pulsenergien, lassen sich sehr regelmässige Abtragsvolumina mit geringer Höhe und geringem Aspektverhältnis erzeugen (Annäherung an Sphäre). Es ist sogar möglich mit hoher numerischer Apertur und Pulsen von sehr kurzer Dauer und geringer Energie gasarme oder sogar gasfreie Schnitte zu erzeugen. Überhitzungen, wie im Stand der Technik erwähnt, treten dabei selbst bei grossen Überlappungen einzelner Laserpulse nicht auf.

Vorzugsweise ist das Abtastmodul eingerichtet, den Brennpunkt wesentlich schneller zu bewegen als die Bewegungsgeschwindigkeit eines menschlichen Auges bei einer Blickrichtungsänderung. Wenn das Auge während der Behandlung nicht mechanisch fixiert wird und sich bewegt, dann kann zwar die Grösse des durch das Abtastmuster definierten Bearbeitungsteilgebiets durch die Augenbewegung leicht verändert werden, doch das Abtastmodul bewegt den Brennpunkt schnell genug, um zu verhindern, dass im Bearbeitungsteilgebiet auf Grund der Augenbewegungen Gewebebrücken verbleiben. Zudem umfasst die Vorrichtung ein Augenüberwachungsmodul zur Bestimmung von Augenbewegungen, und ist eingerichtet, das Positionierungsmodul basierend auf den bestimmten Augenbewegungen für einen entsprechenden Positionierungsausgleich anzusteuern. Da das Positionierungsmodul den Brennpunkt mit einer wesentlich kleineren Frequenz positioniert als das Abtastmodul, wirken sich Augenbewegungen auch entsprechend stärker auf die Positionierung des Brennpunkts an die Ausgangspunkte aus. Durch die Bestimmung der Augenbewegungen, z.B. auf der Basis von lris- oder Venenmuster (auf der Sclera oder er Retina), kann der Einfluss der Augenbewegung jeweils bei der Positionierung des Brennpunkts an einen neuen Ausgangspunkt kompensiert werden. Die hohe Ablenkungsgeschwindigkeit des Abtastmoduls für die Erzeugung von Hohlräumen entsprechend dem Abtastmuster und die Kompensation von Augenbewegungen bei der Positionierung der Ausgangspunkte ermöglichen die refraktive Korrektur des Auges, ohne das Auge dafür am Lichtprojektor fixieren zu müssen. Durch die hohe Anzahl der Bearbeitungsteilgebiete, beispielsweise hundert, mitteln sich Bewegungsartefakte aus dem Abtragergebnis heraus. Die Erzeugung der Vielzahl von getrennten Hohlräumen mit hoher Ablenkungsgeschwindigkeit und die Kompensation der Augenbewegungen bei der Positionierung der Hohlräume ermöglichen somit die refraktive Korrektur des Auges mittels Laserpulsen, ohne dass das Auge und/oder der Patient in irgend einer Form mechanisch mit dem Lasersystem verbunden oder fixiert werden müssen.

Vorzugsweise definiert das Abtastmuster ein Bearbeitungsteilgebiet, dessen Durchmesser kleiner als die Dicke der Hornhaut ist. Wenn die Ausmasse des Bearbeitungsteilgebiets mit aufgelöstem Augengewebe kleiner sind, als die Dicke der Hornhaut, dann ist der an der Hornhautoberfläche ersichtliche Dickenverlust kleiner, als es der Höhe des aufgelösten Augengewebes entsprechen würde, insbesondere, wenn das Bearbeitungsteilgebiet mit dem aufgelösten Augengewebe von der Hornhautoberfläche entfernt ist (beispielsweise mehr als die Hälfte eines Fokusdurchmessers). Über die seitliche Ausdehnung (Durchmesser) und die Tiefenpositionierung eines Bearbeitungsteilgebiets kann dieser Effekt (der beschränkten Dickenreduktion an der Hornhautoberfläche) beeinflusst werden. Somit können durch Bearbeitungsteilgebiete, deren Durchmesser kleiner als die Dicke der Hornhaut ist, Brechkraftänderungen erzielt werden, die kleiner sind, als die der aufgelösten Gewebehöhe entsprechende Brechkraftänderung. Gemäss einer Faustformel für LASIK (Laser In Situ Keratomileusis) entspricht beispielsweise ein Hornhautabtrag von 12µm ungefähr einer Dioptrie. Durch die Auflösung von Augengewebe in kleinen, von der Hornhautoberfläche entfernten Bearbeitungsteilgebieten können somit Korrekturen der Brechkraft vorgenommen werden, die feiner sind, als dies durch eine ausgedehnte Gewebeauflösung der selben Höhe mittels den selben Laserpulsen möglich wäre.

### Kurze Beschreibung der Zeichnungen

Nachfolgend wird eine Ausführung der vorliegenden Erfindung anhand eines Beispieles beschrieben. Das Beispiel der Ausführung wird durch die folgenden beigelegten Figuren illustriert:
Figur 1 a zeigt ein Blockdiagramm, welches schematisch eine ophthalmologische Vorrichtung bei der Behandlung eines Auges mittels eines fokussierten gepulsten Laserstrahls darstellt.
Figur 1b zeigt eine Aufsicht einer durch die ophthalmologische Vorrichtung gemäss einem Abtastmuster bearbeiteten Bearbeitungsfläche.
Figur 2 zeigt ein Flussdiagram, welches den Ablauf bei der refraktiven Korrektur von Augengewebe durch die Gewebeauflösung in einer Vielzahl von voneinander getrennten Bearbeitungsteilgebieten illustriert.
Figur 3a zeigt einen Querschnitt durch ein Segment einer Augenhomhaut, in welcher zur refraktiven Korrektur Augengewebe in einer Vielzahl von voneinander getrennten Bearbeitungsteilgebieten aufgelöst wird.
Figur 3b zeigt eine Aufsicht einer Augenhomhaut, in welcher Gewebe zur refraktiven Korrektur in einer Vielzahl von sich nicht berührenden, in einem ringförmigen Cluster nebeneinander und übereinander liegend angeordneten Bearbeitungsteilgebieten aufgelöst wird.
Figur 3c zeigt einen weiteren Querschnitt durch das Segment der Augenhornhaut, in welcher die von voneinander getrennten Bearbeitungsteilgebiete in äquidistanten, gekrümmten Bearbeitungsflächen angeordnet sind.
Figur 4 zeigt in der Aufsicht die Überlappung der Fokusdurchmesser von mehreren nacheinander folgenden Laserpulsen.

### Wege zur Ausführung der Erfindung

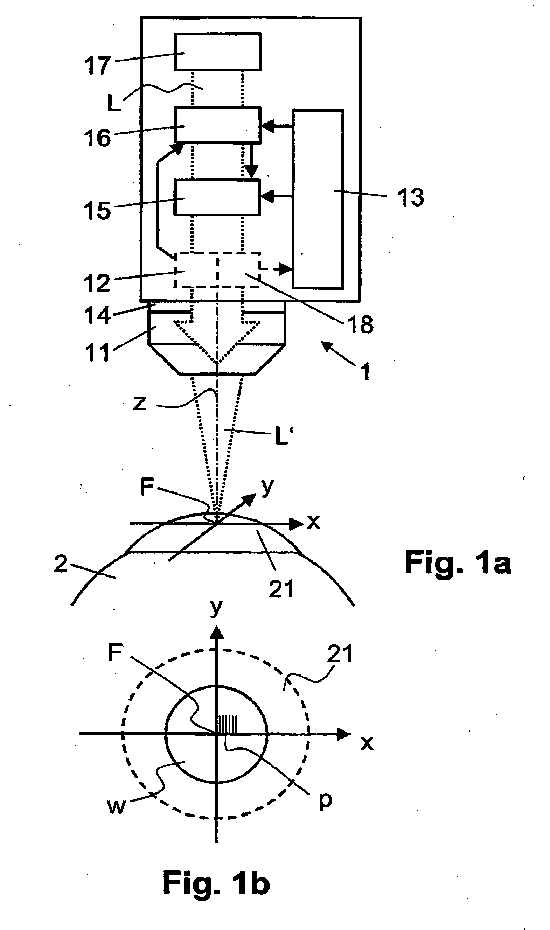

In der Figur 1 a bezeichnet das Bezugszeichen 1 eine ophthalmologische Vorrichtung, respektive eine ophthalmologische Vorrichtungsanordnung, mit einer Laserquelle 17 und einem mit der Laserquelle 17 optisch verbundenen optischen Lichiprojektionsmodul 11 zur Erzeugung und fokussierten Projektion eines gepulsten Laserstrahls L' für die punktuelle Gewebeauflösung in einem Brennpunkt F (Fokus) im Innern des Augengewebes, beispielsweise in der Augenhornhaut 21 (Cornea). Die Laserquelle 17 umfasst insbesondere einen Femtosekundenlaser zur Erzeugung von Femtosekundenlaserpulsen, die Pulsbreiten von typisch 10fs bis 1000fs (1fs=10⁻¹⁵s) aufweisen. Die Laserquelle 17 ist in einem separaten oder in einem mit dem Lichtprojektionsmodul 11 gemeinsamen Gehäuse angeordnet.

Zum besseren Verständnis soll hier angeführt werden, dass die Figur 1a die ophthalmologische Vorrichtung 1 schematisch und vereinfacht darstellt. Zum Beispiel ist in der Figur 1 a nicht präzise wiedergegeben, dass das optische Lichtprojektionsmodul 11 eine hohe numerische Apertur von mindestens 0.3 und vorzugsweise mehr als 0.4 aufweist, dass die ophthalmologische Vorrichtung 1 optional einen Saugring zur Befestigung am Auge 2 aufweist, oder dass die ophthalmologische Vorrichtung 1 optional einen Kantaktkörper (z.B. einen Applanationskörper) zur kontaktbasierten Verformung (z.B. zur Applanierung) des Auges 2 bei der Applikation der ophthalmologischen Vorrichtung 1 umfasst.

Wie in der Figur 1 a schematisch dargestellt ist, umfasst die ophthalmologische Vorrichtung 1 ein Positionierungsmodul 16 und ein Abtastmodul 15, welche im schematisch dargestellten Strahlengang L zwischen Laserquelle 17 und Austritt des Lichtprojektionsmoduls 11 angeordnet sind. Der Fachmann wird verstehen, dass das Positionierungsmodul 16 und das Abtastmodul 15 auch in umgekehrter Reihenfolge angeordnet sein können, als dies in der Figur 1 a dargestellt ist. Das Positionierungsmodul 16 und das Abtastmodul 15 sind kaskadierte Scannermodule, welche den Brennpunkt F positionieren respektive bewegen. Das Positionierungsmodul 16 ist ein wesentlich langsameres Scannermodul als das Abtastmodul 15.

Das Positionierungsmodul 16 ist eingerichtet, den Brennpunkt F an definierte Ausgangspunkte zu positionieren. Das Positionierungsmodul 16 umfasst beispielsweise Bewegungstreiber zum mechanischen Verschieben des Lichtprojektors 11 oder von Teilen des Lichtprojektors 11, zum Beispiel Bewegungstreiber für die laterale Verschiebung von Linsen. Die Bewegungstreiber umfassen beispielsweise ein Antriebselement für eine Vorschubrichtung x und ein Antriebselement für eine zur Vorschubrichtung x senkrechte Abtastrichtung y (siehe Figur 1b), beispielsweise Piezomotoren. In einer Ausführungsvariante umfasst das Positionierungsmodul 16 einen Galvanoscanner zum Ablenken der Laserpulse in der Vorschubrichtung x und/oder in der Abtastrichtung y. Die Koordinaten der Ausgangspunkte werden dem Positionierungsmodul 16 vorzugsweise vom Steuermodul 13 zugeführt, beispielsweise Punkt für Punkt oder als Datei mit (z.B. einer Sequenz von) mehreren Ausgangspunkten zum Speichern im Positionierungsmodul 16.

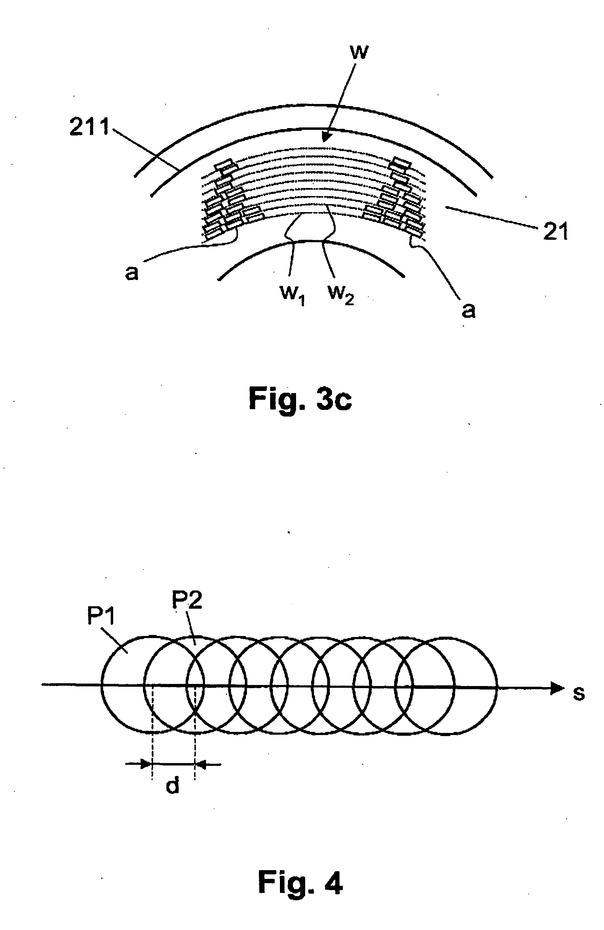

Das Abtastmodul 15 ist eingerichtet, den Brennpunkt F ausgehend vom aktuellen Ausgangspunkt gemäss einem definierten Abtastmuster p zu bewegen (siehe Figur 1b). Um die Laserpulse entsprechend dem vorgegebenen Abtastmuster p zu bewegen, umfasst das Abtastmodul 15 Ablenkungselemente, beispielsweise einen Galvanoscanner, einen resonanten Spiegelscanner, einen akustischen optischen Modulator (AOM), einen Polygonscanner oder einen mikroelektromechanischer Scanner (MEM). In einer Variante umfasst das Abtastmodul 15 zur Positionierung in einer gegenüber der Abtastrichtung y viel langsameren Vorschubrichtung x Bewegungstreiber zum mechanischen Verschieben des Lichtprojektors 11 oder von Teilen des Lichtprojektors 11, d.h. die Bewegung in der langsameren Vorschubrichtung x des Abtastmusters p kann beispielsweise durch das Positionierungsmodul 16 ausgeführt werden. Das Abtastmodul 15 ist entweder fest für ein bestimmtes Abtastmuster p oder für mehrere wählbare Abtastmuster p eingerichtet. Das Abtastmuster p definiert beispielsweise ein rechteckiges, rundes, elliptisches, sternförmiges, spiralförmiges oder Lissajou-Figur-förmiges Bearbeitungsteilgebiet a, und spezifiziert entsprechende Ablenkungen der Laserpulse in der Vorschubrichtung x und in der Abtastrichtung y. Zur refraktiven Korrektur der Augenhornhaut 21 definiert das Abtastmuster p vorzugsweise ein Bearbeitungsteiigebiet a, dessen Durchmesser kleiner als die Dicke der Augenhornhaut 21 ist. Vorzugsweise umfasst das Abtastmodul 15 die Ansteuerung der Ablenkungselemente zur Bewegung des Brennpunkts F gemäss dem Abtastmuster p, der Fachmann wird jedoch verstehen, dass die Ansteuerung auch durch das Steuermodul 13 erfolgen kann. Gegebenenfalls erfolgt die Auswahl eines Abtastmusters p durch das Steuermodul 13, beispielsweise durch entsprechende Auswahlinstruktionen oder Steuersequenzen für das betreffende Auswahlmuster p. Das Abtastmodul 15 ist zudem eingerichtet, die Laserpulse so Abzulenken, dass sich die Fokusdurchmesser P1, P2 von nacheinander folgenden Laserpulsen teilweise überlappen. Wie in der Figur 4 dargestellt ist überlappen sich die Fokusdurchmesser P1, P2 von in der Abtastrichtung s nacheinander folgenden Laserpulsen vorzugsweise um mehr als die Hälfte ihres Durchmessers, das heisst der Abstand d zwischen den Zentren der Fokusdurchmesser P1, P2 ist kleiner als der Radius der Fokusdurchmesser P1, P2. Das Abtastmodul 15 ist eingerichtet, den Brennpunkt F wesentlich schneller zu bewegen als sich das menschliche Auge bei einer Blickrichtungsänderung bewegt. Insbesondere ist das Abtastmodul 15 eingerichtet, die Laserpulse so schnell abzulenken, dass das Augengewebe in einem durch das Abtastmuster definierten Bearbeüungsteilgebiet a ohne darin verbleibende Gewebebrücken aufgelöst wird, auch wenn sich das Auge 2 bewegt. Das gesamte Abtastmuster p für ein Bearbeftungsteilgebiet wird durch das Abtastmodul 15 beispielsweise in 1 ms (Millisekunde) abgefahren. Wenn das Abtastmodul 15 zum Beispiel eingerichtet ist, ein Abtastmuster mit 20KHz in der Vorschubrichtung x abzufahren, können in 1 ms 20 Abtastzeilen in der Abtastrichtung y gefahren werden, bei einem Fokusdurchmesser beispielsweise im Bereich von 5µm bis 20µm wird das Augengewebe in einem Bearbeitungsteilgebiet a mit einem Durchmesser im Bereich von ungefähr 100µm bis 400µm aufgelöst (bei Überlappung der Fokusdurchmesser P1, P2 in der Vorschubrichtung x wird dieser Wert entsprechend reduziert). Eine eventuelle Augenbewegung in x Richtung wird diesen Bereich geringfügig strecken oder stauchen, wobei ein durchgehender Schnitt immer gewährleistet bleibt.

Eine ophthalmologische Vorrichtung mit einer mechanischen Bewegung des Lichtprojektors und einer Überlagerung einer zusätzlichen feinen Bewegung des Brennpunkts F mittels optischer Microscans wird in der per Referenz miteingeschlossenen EP 1 486 185 beschrieben. In der noch nicht veröffentlichten Europäischen Patentanmeldung Nr. 05 405 376 werden ein Scannermodul zur Ablenkung des gepulsten Lichtstrahls für die zusätzliche feine Bewegung, sowie ein optisches Übertragungssystem zur Übertragung der abgelenkten Femtosekundenlaserpulse vom Scannermodul zum Lichtprojektor 11 und zur Überlagerung der abgelenkten Femtosekundenlaserpulse auf die Bewegung des Lichtprojektors 11 beschrieben.

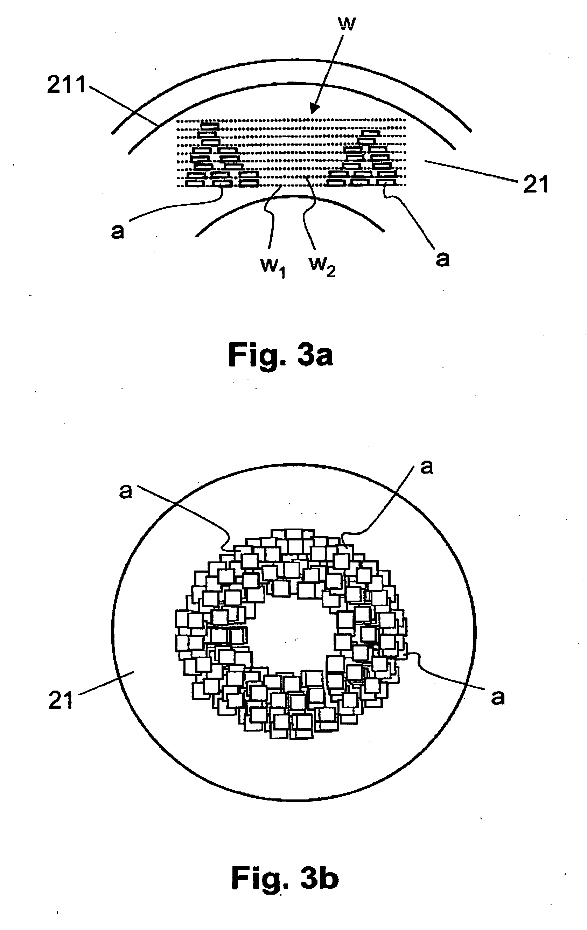

Wie in der Figur 1 a schematisch dargestellt ist, umfasst die ophthalmologische Vorrichtung 1 zudem ein Tiefenpositionierungsmodul 14 zum Verschieben des Brennpunkts F entlang einer Projektionsachse z des Lichtprojektors 11, beispielsweise senkrecht zu einer durch die Vorschubrichtung x und die Abtastrichtung y aufgespannten Bearbeitungsfläche, insbesondere eine Bearbeitungsebene w. Zum Verschieben des Brennpunkts F umfasst das Tiefenpositionierungsmodul 14 vorzugsweise eine bewegliche Fokussierungslinse und ein damit gekoppeltes Antriebselement. In einer alternativen Variante wird der Lichtprojektor 11 zur Tiefeneinstellung mechanisch bewegt. Wie im schematischen Querschnitt der Figur 3a illustriert wird, werden durch die Tiefeneinstellung des Brennpunkts F unterschiedlich tiefe Fokalebenen respektive Fokalflächen definiert, welche als Bearbeitungsebenen respektive Bearbeitungsflächen w, w₁, w₂ dienen, auf denen das Augengewebe, beispielsweise die Augenhornhaut 21, jeweils in mehreren voneinander getrennten, durch Abtastmuster p definierten Bearbeitungsteilgebieten a aufgelöst wird. Zusätzlich zu den in der Figur 3a dargestellten ebenen Bearbeitungsflächen w, w₁, w₂ können die Bearbeitungsflächen w, w₁, w₂ durch entsprechend gesteuerte Tiefeneinstellung des Brennpunkts F oder durch die Verwendung im Lichtprojektor 11 von Objektiven mit sphärischen Bildfeldern auch gekrümmt (konkav, konvex) ausgestaltet werden, wie dies in der Figur 3c dargestellt ist. Durch die Gewebeauflösung entsteht in jedem der Bearbeitungsteilgebiete a ein Hohlraum, der jeweils von den anderen Hohlräumen, sowohl auf der selben Bearbeitungsfläche w, w₁, w₂ als auch auf benachbarten, übereinander liegenden Bearbeitungsflächen w₁, w₂, durch Gewebebrücken getrennt ist. Vorzugsweise werden die Bearbeitungsteilgebiete a von der Hornhautoberfläche entfernt angeordnet, vorzugsweise unter der Bowmanschen Membran 211 der Hornhaut 21. Die Figur 3b illustriert in der Aufsicht ein Beispiel von einer Vielzahl von sich nicht berührenden Bearbeitungsteilgebieten a (beispielsweise hundert oder mehr), die in einem ringförmigen Cluster nebeneinander und übereinander liegend angeordnet sind, zur Bildung einer Vielzahl von voneinander getrennten, nicht zusammenhängenden Hohlräumen, welche kollabieren und dadurch die Hornhautkrümmung für eine gewünschte refraktive Korrektur der Augenhornhaut 21 geeignet ändern.

In einer weiteren Ausführungsvariante umfasst die ophthalmologische Vorrichtung 1 einen Wellenfrontdetektor 18 zum Bestimmen eines Wellenfrontverlaufs eines durch das Auge 2 reflektierten Lichtbündels. Das reflektierte Lichtbündel ist ein zusätzlicher Referenzlichtstrahl, der vom Augenhintergrund reflektiert und dem Wellenfrontdetektor 18 mittels optischer Elemente zugeführt wird. Der Wellenfrontdetektor ist beispielsweise als Shack-Hartmann-Sensor ausgeführt, beispielsweise nach US 2003/0038921, oder als Interferometer, z.B. als Shearing-Interferometer. Weitere mögliche Ausführungsformen des Wellenfrontdetektors sind in Jos. J. Rozena, Dirk E.M. Van Dyck, Marie-José Tassignon, "Clinical comparison of 6 aberrometers. Part 1: Technical specifications", J Cataract Refract Surg, Volume 31, Juni 2005, Seiten 1114-1127, beschrieben. Zur Rückmeldung des bestimmten Wellenfrontverlaufs ist der Wellenfrontdetektor 18 mit dem Steuermodul 13 verbunden. Das Steuermodul 13 ist eingerichtet, basierend auf dem bestimmten Wellenfrontverlauf die aktuell erreichte refraktive Korrektur der Augenhornhaut 21 zu bestimmen und darauf basierend die örtliche Verteilung weiterer Bearbeitungsteilgebiete a respektive die Ausgangspunkte für entsprechende Abtastmuster p zu bestimmen, um die gewünschte refraktive Korrektur der Augenhornhaut 21 zu erreichen. Je nach Ausführungsvariante wird die Bestimmung des Wellenfrontverlaufs und der Ausgangspunkte für weitere Bearbeitungsteilgebiete a zu unterschiedlichen Zeitpunkten durchgeführt, beispielsweise periodisch nach einem vorgegebenen Zeitplan, nach der Auflösung des Augengewebes in sämtlichen geplanten Bearbeitungsteilgebieten a auf einer Bearbeitungsfläche w, w₁, w₂, nach der Bearbeitung sämtlicher geplanter Bearbeftungsteilgebiete a und/oder nach dem Empfang eines über eine Benutzerschnittstelle eingegebenen Instruktionssignals.

In einer weiteren Ausführungsvariante umfasst die ophthalmologische Vorrichtung 1 ein Augenüberwachungsmodul 12 (ein so genannter "Eye Tracker") zur Bestimmung von Augenbewegungen. Das Augenüberwachungsmodul 12 umfasst beispielsweise eine Kamera, beispielsweise eine CCD-Kamera (Charged Coupled Device) und eine Beleuchtungseinrichtung (z.B. LEDs), zur Erfassung einer Aufsicht des Auges 2, sowie Verarbeitungsmittel zur Bestimmung des Iris- oder Venenmusters (auf der Sclera oder der Retina) in der Aufsicht und zur Bestimmung von Augenbewegungen basierend auf relativen Verschiebungen des Iris- oder Venenmusters. Die Verarbeitungsmittel sind als programmiertes Logikmodul mittels Software und/oder Hardware ausgeführt und sind in einer Variante im Steuermodul 13 angeordnet. Detektierte Augenbewegungen werden vom Augenüberwachungsmodul 12 laufend an das Positionierungsmodul 16 oder an das Steuermodul 13 übertragen, beispielsweise als Relativwerte bezüglich einer definierten Referenzposition des Auges 2 oder als Blickrichtungswerte des Auges 2. Das Positionierungsmodul 16 respektive das Steuermodul 13 ist eingerichtet, bei der Positionierung des Brennpunkts F in einen Ausgangspunkt Bewegungen des Auges 2 basierend auf den bestimmten Augenbewegungen auszugleichen. Das Positionierungsmodul 16 korrigiert die Koordinaten vorgegebener Ausgangspunkte basierend auf den detektierten Augenbewegungen oder das Steuermodul 13 liefert dem Positionierungsmodul 16 Ausgangspunkte, deren Koordinaten entsprechend den Augenbewegungen angepasst sind.

Das Steuermodul 13 ist vorzugsweise als programmiertes Logikmodul mittels Software und/oder Hardware ausgeführt. Das Steuermodul 13 ist zur Übertragung von Steuersignalen und/oder Steuerdaten mit dem Positionierungsmodul 16 und dem Abtastmodul 15 verbunden. Je nach Ausführungsvariante ist das Steuermodul 13 zum Empfang von Rückmeldungen oder Datenwerten über Augenbewegungen mit dem Wellenfrontdetektor 18 und/oder dem Augenüberwachungsmodul 12 verbunden. Das Steuermodul 13 ist in einem separaten oder in einem mit dem Lichtprojektionsmodul 11 gemeinsamen Gehäuse angeordnet. Das Steuermodul 13 ist eingerichtet, für eine gewünschte refraktive Korrektur des Auges 2, insbesondere der Augenhornhaut 21, die örtliche Verteilung der dazu nötigen Bearbeitungsteilgebiete a im Innern des Auges 2 zu bestimmen, das heisst die Anzahl der Bearbeitungsteilgebiete a, die jeweils zugeordneten Ausgangspunkte (in mehreren Bearbeitungsflächen) und in einer Variante auch das entsprechende Abtastmuster p respektive die durch das Abtastmuster p definierte Grösse, Form und/oder Ausrichtung der Bearbeitungsteilgebiete a. In einer Variante ist das Steuermodul 13 eingerichtet, den Wellenfrontverlauf eines durch das Auge 2 reflektierten Lichtbündels und damit die aktuelle Brechkraft der Augenhornhaut 21 mittels des Wellenfrontdetektors 18 zu ermitteln, und darauf basierend die örtliche Verteilung der Bearbeitungsteilgebiete a im Innern des Auges 2 zu bestimmen.

Das Steuermodul 13 bestimmt die Anzahl und örtliche Verteilung der Bearbeitungsteilgebiete a beispielsweise auf Grund einer Tabelle. Die Tabelle ordnet verschiedenen refraktiven Korrekturwerten (und Korrekturarten) jeweils eine Anzahl und örtliche Verteilung der Bearbeifungsteügebiete a zu. In einer weiteren Variante bestimmt das Steuermodul 13 die Anzahl und örtliche Verteilung der Bearbeitungsteilgebiete a auf Grund eines Modells des zu behandelnden Augengewebes, z.B. ein Augenhornhautmodel und Informationen darüber, wie das Auge 2 abbildet, bei vorgegebener Grösse und Form der Bearbeitungsteilgebiete a und bei vorgegebenen vertikalen und horizontalen Mindestabständen einzelner Bearbeitungsteilgebiete a. Die Angaben über die Anzahl und die örtliche Verteilung der Bearbeitungsteilgebiete a können auch von einer externen Einheit an das Steuermodul 13 übermittelt werden. Die örtliche Verteilung der Bearbeitungsteilgebiete a erfolgt so, dass die bei der Auflösung des Augengewebes in den Bearbeitungsteilgebieten a entstehenden Hohlräume sowohl auf der selben Bearbeitungsfläche w, w₁, w₂ als auch in benachbarten, übereinander liegenden Bearbeitungsflächen w₁, w₂ jeweils durch Gewebebrücken voneinander getrennt sind. Die Anzahl der Bearbeitungsteilgebiete a kann in einer Variante auch aus einem Abtragsvolumen bestimmt werden, dass für eine spezifizierte refraktive Korrektur bestimmt oder durch den Benutzer eingegeben wird. Die örtliche Anordnung der Bearbeitungsteilgebiete a wird durch die Korrekturart bestimmt, beispielsweise muss bei einer Myopie die Augenhornhaut 21 durch zentralisierte Abtragung abgeflacht werden, wohingegen bei einer Hyperopie die Krümmung der Hornhautoberfläche durch ringförmig umlaufende Abtragung steiler ausgestaltet werden muss.

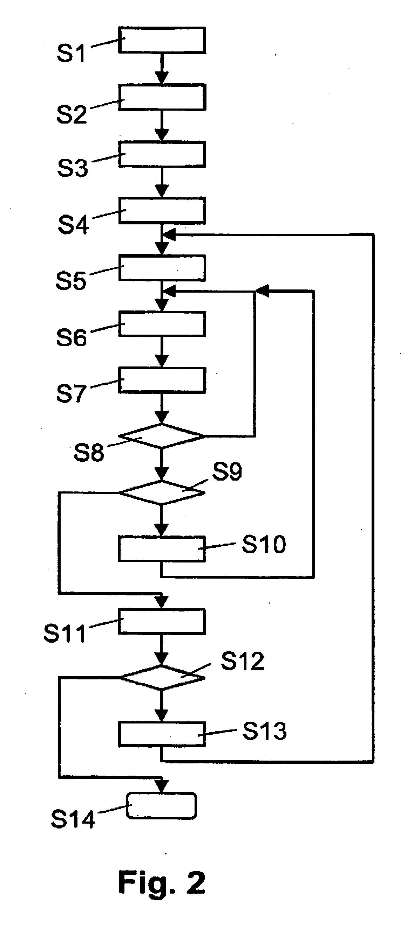

In den nachfolgenden Abschnitten wird mit Bezug zu der Figur 2 der durch das Steuermodul 13 gesteuerte Ablauf bei der refraktiven Korrektur eines Auges 2 beschrieben.

Im Schritt S1 bestimmt das Steuermodul 13 die gewünschte refraktive Korrektur des Auges 2. Der Sollwert der refraktiven Korrektur wird beispielsweise über eine Benutzerschnittstelle eingegeben und im Steuermodul 13 erfasst.

Im Schritt S2 bestimmt das Steuermodul 13 die örtliche Verteilung der Bearbeitungsteilgebiete a, um die gewünschte refraktive Korrektur zu erreichen.

Im optionalen Schritt S3 übermittelt das Steuermodul 13 die Ausgangspunkte für die Abtastmuster p der Bearbeitungsteilgebiete a an das Positionierungsmodul 16 beispielsweise als Sequenz von Ausgangspunkten, geordnet nach abnehmender Tiefe der Bearbeitungsfläche w, w₁, w₂. In einer Variante sind den verschiedenen Ausgangspunkten auch Identifizierungselemente von unterschiedlichen Abtastmustem p zugeordnet. Gegebenenfalls werden auch Steuerwerte für unterschiedliche Abtastmuster p an das Abtastmodul 15 übermittelt.

Im Schritt S4 wird die Behandlung des Auges 2 durch ein über die Benutzerschnittstelle eingegebenes Startsignal gestartet.

Im Schritt S5 wird der Brennpunkt F auf die tiefstgelegene Bearbeitungsfläche w₁ positioniert. Die entsprechende Ansteuerung des Tiefenpositionierungsmoduls 14 erfolgt vorzugsweise durch das Steuermodul 13.

Im Schritt S6 positioniert das Positionierungsmodul 16 den Brennpunkt F in der aktuellen Bearbeitungsfläche w, w₁, w₂ an einen noch nicht verwendeten Ausgangspunkt. Die Positionierung erfolgt gemäss Vorgabe des Ausgangspunkt durch das Steuermodul 13 oder gemäss einer vorher im Positionierungsmodul 16 gespeicherten Sequenz von Ausgangspunkten. Bei der Positionierung werden auch die dauernd überwachten Augenbewegungen berücksichtigt und entweder im Steuermodul 13 oder im Positionierungsmodul 16 kompensiert.

Im Schritt S7 bewegt das Abtastmodul 15 den Brennpunkt F in der aktuellen Bearbeitungsfläche w, w₁, w₂, ausgehend vom aktuellen Ausgangspunkt entsprechend dem Abtastmuster p, das dem aktuellen Ausgangspunkt zugeordnet ist. Das zu verwendende Abtastmuster p ist beispielsweise für die gesamte Behandlung unverändert oder wird durch das Steuermodul 13 oder das Positionierungsmodul 16, beispielsweise bei der Übermittlung eines Synchronisationssignals, mittels eines Identifizierungselements bestimmt.

Im Schritt S8 überprüft das Steuermodul 13, ob sämtliche Ausgangspunkte der aktuellen Bearbeitungsfläche w, w₁, w₂ bereits bearbeitet wurden. Falls zu bearbeitende Ausgangspunkte verbleiben, erfolgt die Positionierung des nächsten Ausgangspunkt im Schritt S6. Falls auf der aktuellen Bearbeitungsfläche w, w₁, w₂ alle zugeordneten Ausgangspunkte bearbeitet wurden, fährt das Steuermodul im Schritt S9 fort.

Im Schritt S9 überprüft das Steuermodul 13, ob sämtliche Bearbeitungsflächen w, w₁, w₂ bereits bearbeitet wurden. Falls zu bearbeitende Bearbeitungsflächen w, w₁, w₂ verbleiben, wird der Brennpunkt F im Schritt S10 auf die nächst höher gelegene, äquidistante (z.B. parallele) Bearbeitungsfläche w, w₂ positioniert, auf der Ausgangspunkte zu bearbeiten sind, und das Positionierungsmodul 16 fährt im Schritt S6 mit der Positionierung des nächsten Ausgangspunkts fort. Ansonsten, wenn sämtliche Bearbeitungsflächen w, w₁, w₂ mit zu bearbeitenden Ausgangspunkten bereits bearbeitet wurden, fährt das Steuermodul im Schritt S11 fort.

Im optionalen Schritt S11 bestimmt der Wellenfrontdetektor 18 den Wellenfrontverlauf des Auges 2 und übermittelt diesen an das Steuermodul 13 (ohne Schritte S11, S12, S13 endet das Verfahren im Schritt S14).

Im Schritt S12 bestimmt das Steuermodul 13 auf Grund des Wellenfrontverlaufs, ob die gewünschte refraktive Korrektur erreicht wurde. Falls die gewünschte Korrektur erreicht wurde, beendet das Steuermodul 13 das Verfahren im Schritt S14, beispielsweise mit einer Erfolgsmeldung über die Benutzerschnittstelle. Andernfalls, wenn die gewünschte refraktive Korrektur noch nicht erreicht wurde, fährt das Steuermodul im Schritt S13 fort.

Im Schritt S13 bestimmt das Steuermodul 13, vorzugsweise nach erfolgter Rückmeldung und Bestätigung über die Benutzerschnittstelle, die örtliche Verteilung zusätzlicher Bearbeitungsteilgebiete a, die zum Erreichen der gewünschten refraktiven Korrektur zu bearbeiten sind. Die Bearbeitung der weiteren Bearbeitungsteilgebiete a erfolgt im Schritt S5, gegebenenfalls nach der Übermittlung der zusätzlichen Ausgangspunkte an das Positionierungsmodul 16.

## Patentansprüche

1. Ophthalmologische Vorrichtung (1) für eine refraktive Korrektur eines menschlichen Auges (2), umfassend:
einen Lichtprojektor (11) zur Projektion von Laserpulsen (L') auf einen Brennpunkt (F) im Innern der Hornhaut (21) des Auges (2) für eine Auflösung von Augengewebe der Hornhaut (21),
ein Positionierungsmodul (16) zur Positionierung des Brennpunkts (F) an unterschiedliche Ausgangspunkte im Innern der Hornhaut (21),
ein Abtastmodul (15) zur Bewegung des Brennpunkts (F) ausgehend von jeweils einem der Ausgangspunkte gemäss einem definierten Abtastmuster (p) zur Bearbeitung eines Bearbeitungsteilgebiets (a) im Innern der Hornhaut (21) mit jeweils mehreren Laserpulsen (L'),
wobei das Positionierungsmodul (16) und das Abtastmodul (15) kaskadierte Scannermodule sind, und das Positionierungsmodul (16) ein wesentlich langsameres Scannermodul als das Abtastmodul (15) ist, und
ein Augenüberwachungsmodul (12) zur Bestimmung von Augenbewegungen,
wobei das Positionierungsmodul (16) eingerichtet ist, bei der Positionierung des Brennpunkts (F) an einen Ausgangspunkt jeweils Bewegungen des Auges (2) basierend auf den bestimmten Augenbewegungen auszugleichen,
wobei das Abtastmuster (p) und die Ausgangspunkte so definiert sind, dass das Augengewebe im Innern der Hornhaut (21) in mehreren durch Gewebebrücken voneinander getrennten Bearbeitungsteilgebieten (a) jeweils durch mehrere gemäss dem Abtastmuster (p) bewegte Laserpulse (L1') als Hohlraum aufgelöst wird, wobei der Hohlraum von anderen Hohlräumen durch Gewebebrücken getrennt ist, und
wobei das Abtastmodul (15) eingerichtet ist, die Laserpulse (L') so schnell abzulenken, dass der Brennpunkt (F) wesentlich schneller bewegt wird als sich das Auge (2) bei einer Blickrichtungsänderung bewegt, und das Augengewebe für die Erzeugung der Hohlräume jeweils in den durch das Abtastmuster (p) definierten Bearbeitungsteilgebieten (a) durch mehrere gemäss dem Abtastmuster (p) bewegte Laserpulse (L1') ohne darin verbleibende Gewebebrücken aufgelöst wird, auch wenn sich das Auge (2) bewegt.

2. Vorrichtung (1) nach Anspruch 1, **dadurch gekennzeichnet, dass** das Positionierungsmodul (16) eingerichtet ist, den Brennpunkt (F) jeweils an Ausgangspunkte auf einer ersten Bearbeitungsfläche (w₁) zu positionieren, dass das Abtastmodul (15) eingerichtet ist, den Brennpunkt (F) in der ersten Bearbeitungsfläche (w₁) zu bewegen, und dass das Tiefenpositionierungsmodul (14) eingerichtet ist, den Brennpunkt (F) entlang der Projektionsachse (z) des Lichtprojektors (11) in eine zur ersten Bearbeitungsfläche (w₁) äquidistante zweite Bearbeitungsfläche (w₂) zu verschieben, so dass der Brennpunkt (F) in der zweiten Bearbeitungsflächen (w₂) an unterschiedliche Ausgangspunkte positionierbar und ausgehend von jeweils einem der Ausgangspunkte gemäss dem Abtastmuster (p) bewegbar ist.

3. Vorrichtung (1) nach einem der Ansprüche 1 oder 2, **gekennzeichnet durch** ein Steuermodul (13), welches eingerichtet ist, entsprechend einer gewünschten refraktiven Korrektur des Auges (2) eine Anzahl der Bearbeitungsteilgebiete (a) und die Ausgangspunkte für eine örtliche Verteilung der Bearbeitungsteilgebiete (a) in mehreren Bearbeitungsflächen (w, w₁, w₂) im Innern des Auges (2) zu bestimmen.

4. Vorrichtung (1) nach Anspruch 3, **dadurch gekennzeichnet, dass** das Steuermodul (13) zudem eingerichtet ist, unterschiedliche Abtastmuster (p) für unterschiedlich grosse Bearbeitungsteilgebiete (a) zu wählen.

5. Vorrichtung (1) nach einem der Ansprüche 3 oder 4, **dadurch gekennzeichnet, dass** die Vorrichtung (1) einen Wellenfrontdetektor (18) umfasst zum Bestimmen eines Wellenfrontverlaufs eines durch das Auge (2) reflektierten Lichtbündels, und dass die Vorrichtung (1) ein Steuermodul (13) umfasst, welches eingerichtet ist, die Ausgangspunkte basierend auf dem bestimmten Wellenfronlverlauf festzulegen.

6. Vorrichtung (1) nach einem der Ansprüche 3 bis 5, **dadurch gekennzeichnet, dass** das Steuermodul (13) zudem eingerichtet ist, das Tiefenpositionierungsmodul (14) so zu steuern, dass beim Verschieben des Brennpunkts (F) eine Mindestdistanz zwischen den Bearbeitungsflächen (w, w₁, w₂) eingehalten wird, wobei die Mindestdistanz so definiert ist, dass in äquidistanten Bearbeitungsflächen (w, w₁, w₂) über einander liegende Bearbeitungsteilgebiete (a) durch Gewebebrücken voneinander getrennt sind.

7. Vorrichtung (1) nach einem der Ansprüche 1 bis 6, **dadurch gekennzeichnet, dass** das Abtastmodul (15) eingerichtet ist, nacheinander folgende Laserpulse (L') so zu positionieren, dass sich ihre Fokusdurchmesser (P1, P2) teilweise überlappen.

8. Vorrichtung (1) nach Anspruch 7, **dadurch gekennzeichnet, dass** sich ihre Fokusdurchmesser (P1, P2) mindestens bis zur Hälfte ihres Durchmessers überlappen.

9. Vorrichtung (1) nach einem der Ansprüche 1 bis 8, **dadurch gekennzeichnet, dass** das Abtastmodul (15) eingerichtet ist, den Brennpunkt (F) wesentlich schneller zu bewegen als eine Bewegungsgeschwindigkeit eines menschlichen Auges (2) bei einer Blickrichtungsänderung.

10. Vorrichtung (1) nach einem der Ansprüche 1 bis 9, **dadurch gekennzeichnet, dass** die Vorrichtung (1) ein Augenüberwachungsmodul (12) umfasst zur Bestimmung von Augenbewegungen, und dass die Vorrichtung (1) eingerichtet ist, das Positionierungsmodul (16) basierend auf den bestimmten Augenbewegungen für einen entsprechenden Positionierungsausgleich anzusteuern.

11. Vorrichtung (1) nach einem der Ansprüche 1 bis 10, **dadurch gekennzeichnet, dass** das Abtastmuster (p) jeweils ein Bearbeitungsteilgebiet (a) mit einer Form aus rechteckig, rund, elliptisch, sternförmig, spiralförmig und Lissajou-Figur-förmig definiert.

12. Vorrichtung (1) nach einem der Ansprüche 1 bis 11, **dadurch gekennzeichnet, dass** das Abtastmuster (p) ein Bearbeitungsteilgebiet (a) definiert, dessen Durchmesser kleiner als die Dicke der Hornhaut (21) ist.

13. Vorrichtung (1) nach einem der Ansprüche 1 bis 11, **dadurch gekennzeichnet, dass** das Abtastmodul (15) zum Ablenken der Laserpulse mindestens eines umfasst aus Galvanoscanner, resonanter Spiegelscanner, akustischer optischer Modulator, Polygonscanner und mikroelektromechanischer Scanner.

14. Vorrichtung (1) nach einem der Ansprüche 1 bis 13, **dadurch gekennzeichnet, dass** das Positionierungsmodul (16) Bewegungstreiber umfasst zum mechanischen Verschieben von mindestens Teilen des Lichtprojektors (11).

15. Vorrichtung (1) nach einem der Ansprüche 1 bis 14, **dadurch gekennzeichnet, dass** das Positionierungsmodul (16) einen Galvanoscanner umfasst zum Ablenken der Laserpulse.

16. Vorrichtung (1) nach einem der Ansprüche 1 bis 15, **dadurch gekennzeichnet, dass** der Lichtprojektor (11) eine numerische Apertur von über 0.3 aufweist.

## Claims

1. Ophthalmological apparatus (1) for a refractive correction of a human eye (2), comprising:
a light projector (11) for projecting laser pulses (L') on to a focal point (F) in the interior of the cornea (21) of the eye (2) for breaking down eye tissue of the cornea (21),
a positioning module (16) for positioning the focal point (F) at different starting points in the interior of the cornea (21),
a scanning module (15) for moving the focal point (F) starting from, in each case, one of the starting points in accordance with a defined scanning pattern (p) for the treatment of a treatment subarea (a) in the interior of the cornea (21) with the aid, in each case, of a number of laser pulses (L'),
the positioning module (16) and the scanning module (15) being cascaded scanner modules, and the positioning module (16) being a substantially slower scanner module than the scanning module (15), and
an eye tracking module (12) for determining eye movements,
the positioning module (16) being set up to compensate movements of the eye (2) on the basis of the determined eye movements in each case when positioning the focal point (F) at a starting point,
the scanning pattern (p) and the starting points being defined such that the eye tissue in the interior of the cornea (21) is respectively broken down as a cavity in a number of treatment subareas (a) mutually separated by tissue bridges by means of a number of laser pulses (L1') moving in accordance with the scanning pattern (p), the cavity being separated from other cavities by tissue bridges, and
the scanning module (15) being set up to deflect the laser pulses (L') so quickly that the focal point (F) is moved much more quickly than the eye (2) when changing the direction of view, and in order to produce the cavities the eye tissue is respectively broken down in the treatment subareas (a) defined by the scanning pattern (p) by means of a number of laser pulses (L1') moved in accordance with the scanning pattern (p) without tissue bridges remaining therein, even when the eye (2) moves.

2. Apparatus (1) according to Claim 1, **characterized in that** the positioning module (16) is set up to position the focal point (F) respectively at starting points on a first treatment surface (w₁), **in that** the scanning module (15) is set up to move the focal point (F) on the first treatment surface (w₁), and **in that** the depth positioning module (14) is set up to displace the focal point (F) along the projection axis (z) of the light projector (11) onto a second treatment surface (w₂) equidistant from the first treatment surface (w₁) such that the focal point (F) can be positioned on the second treatment surface (w₂) at different starting points and can be moved in accordance with the scanning pattern (p) starting from one of the starting points in each case.

3. Apparatus (1) according to either of Claims 1 and 2, **characterized by** a control module (13) which is set up to determine in accordance with a desired refractive correction of the eye (2) a number of the treatment subareas (a) and the starting points for a spatial distribution of the treatment subareas (a) on a number of treatment surfaces (w, w₁, w₂) in the interior of the eye (2).

4. Apparatus (1) according to Claim 3, **characterized in that** the control module (13) is further set up to select different scanning patterns (p) for treatment subareas (a) of different size.

5. Apparatus (1) according to either of Claims 3 and 4, **characterized in that** the apparatus (1) comprises one wavefront detector (18) for determining a wavefront profile of a light bundle reflected by the eye (2) and **in that** the apparatus (1) comprises a control module (13) which is set up to fix the starting points on the basis of the determined wavefront profile.

6. Apparatus (1) according to one of Claims 3 to 5, **characterized in that** the control module (13) is further set up to control the depth positioning module (14) such that a minimum distance between the treatment surfaces (w, w₁, w₂) is observed upon displacement of the focal point (F), the minimum distance being defined such that treatment subareas (a) superimposed on equidistant treatment surfaces (w, w₁, w₂) are separated from one another by tissue bridges.

7. Apparatus (1) according to one of Claims 1 to 6, **characterized in that** the scanning module (15) is set up to position consecutive laser pulses (L') such that their focal diameters (P1, P2) partially overlap.

8. Apparatus (1) according to Claim 7, **characterized in that** their focal diameters (P1, P2) overlap at least as far as half their diameter.

9. Apparatus (1) according to one of Claims 1 to 8, **characterized in that** the scanning module (15) is set up to move the focal point (F) much more quickly than a speed of movement of a human eye (2) when changing direction of view.

10. Apparatus (1) according to one of Claims 1 to 9, **characterized in that** the apparatus (1) comprises an eye tracking module (12) for determining eye movements, and **in that** the apparatus (1) is set up to drive the positioning module (16) for an appropriate positioning compensation on the basis of the determined eye movements.

11. Apparatus (1) according to one of Claims 1 to 10, **characterized in that** the scanning pattern (p) respectively defines a treatment subarea (a) whose shape is selected as rectangular, round, elliptical, that of a star, that of a spiral or that of a Lissajou figure.

12. Apparatus (1) according to one of Claims 1 to 11, **characterized in that** the scanning pattern (p) defines a treatment subarea (a) whose diameter is smaller than the thickness of the cornea (21).

13. Apparatus (1) according to one of Claims 1 to 11, **characterized in that** the scanning module (15) for deflecting the laser pulses comprises at least one of the following: a galvanoscanner, resonant mirror scanner, acoustic optical modulator, polygonal scanner and microelectromechanical scanner.

14. Apparatus (1) according to one of Claims 1 to 13, **characterized in that** the positioning module (16) comprises movement drivers for mechanically displacing at least parts of the light projector (11).

15. Apparatus (1) according to one of Claims 1 to 14, **characterized in that** the positioning module (16) comprises a galvanoscanner for deflecting the laser pulses.

16. Apparatus (1) according to one of Claims 1 to 15, **characterized in that** the light projector (11) has a numerical aperture above 0.3.

## Revendications

1. Dispositif ophtalmologique (1) pour la correction réfractive d'un oeil humain (2), comprenant
- un projecteur de lumière (11) pour la projection d'impulsions laser (L') sur un foyer (F) à l'intérieur de la cornée (21) de l'oeil (2) pour une désagrégation du tissu oculaire de la cornée (21),
- un module de positionnement (16) pour positionner le foyer (F) à différents points de repère à l'intérieur de la cornée (21),
- un module de balayage (15) pour déplacer le foyer (F) chaque fois à partir d'un des points de repère selon un motif de balayage défini (p) pour le traitement d'une zone de traitement (a) à l'intérieur de la cornée (21) chaque fois avec plusieurs impulsions laser (L'),
- dans lequel le module de positionnement (16) et le module de balayage (15) sont des modules de scannage en cascade, et le module de positionnement (16) est un module de scannage nettement plus lent que le module de balayage (15), et
- un module de surveillance de l'oeil (12) pour déterminer des mouvements de l'oeil,
- dans lequel le module de positionnement (16) est conçu de façon à compenser chaque fois des mouvements de l'oeil (2) lors du positionnement du foyer (F) à un point de repère, en se basant sur les mouvements de l'oeil déterminés,
- dans lequel le motif de balayage (p) et les points de repère sont définis de telle manière que le tissu oculaire à l'intérieur de la cornée (21) soit désagrégé dans plusieurs zones de traitement (a) séparées l'une de l'autre par des ponts de tissu, chaque fois par plusieurs impulsions laser (L1') déplacées selon le motif de balayage (p) sous la forme d'une cavité, dans lequel la cavité est séparée des autres cavités par des ponts de tissu, et
- dans lequel le module de balayage (15) est conçu de façon à dévier les impulsions laser (L') avec une vitesse telle que le foyer (F) soit déplacé nettement plus rapidement que l'oeil (2) ne se déplace lors d'un changement de direction du regard, et le tissu oculaire est désagrégé sans qu'il y subsiste des ponts de tissu lors de la production des cavités, chaque fois dans les zones de traitement (a) définies par le motif de balayage (p), au moyen d'impulsions laser (L1') déplacées selon le motif de balayage (p), même lorsque l'oeil (2) se déplace.

2. Dispositif (1) selon la revendication 1, **caractérisé en ce que** le module de positionnement (16) est conçu de façon à positionner le foyer (F) chaque fois à des points de repère sur une première face de traitement (w₁), **en ce que** le module de balayage (15) est conçu de façon à déplacer le foyer (F) dans la première face de traitement (w₁), et **en ce que** le module de positionnement en profondeur (14) est conçu de façon à déplacer le foyer (F) le long d'un axe de projection (z) du projecteur de lumière (11) dans une deuxième face de traitement (w₂) équidistante de la première face de traitement (w₁), de telle manière que le foyer (F) puisse être positionné dans la deuxième face de traitement (w₂) à différents points de repère et puisse être déplacé à partir d'un des points de repère selon le motif de balayage (p).

3. Dispositif (1) selon l'une quelconque des revendications 1 ou 2, **caractérisé par** un module de commande (13), qui est conçu de façon à déterminer, selon une correction réfractive désirée de l'oeil (2), un nombre des zones partielles de traitement (a) et les points de repère pour une répartition locale des zones partielles de traitement (a) dans plusieurs faces de traitement (w, w₁, w₂) à l'intérieur de l'oeil (2).

4. Dispositif (1) selon la revendication 3, **caractérisé en ce que** le module de commande (13) est en outre conçu de façon à choisir différents motifs de balayage (p) pour des zones partielles de traitement (a) de grandeur différente.

5. Dispositif (1) selon l'une quelconque des revendications 3 ou 4, **caractérisé en ce que** le dispositif (1) comprend un détecteur de front d'onde (18) pour déterminer un profil de front d'onde d'un faisceau lumineux réfléchi à travers l'oeil (2), et **en ce que** le dispositif (1) comprend un module de commande (13), qui est conçu de façon à fixer les points de repère en se basant sur le profil de front d'onde déterminé.

6. Dispositif (1) selon l'une quelconque des revendications 3 à 5, **caractérisé en ce que** le module de commande (13) est en outre conçu de façon à commander le module de positionnement en profondeur (14) de telle manière que, lors du déplacement du foyer (F), on respecte une distance minimale entre les faces de traitement (w, w₁, w₂), dans lequel la distance minimale est définie de telle manière que des zones partielles de traitement (a) situées l'une au-dessus de l'autre dans des faces de traitement équidistantes (w, w₁, w₂) soient séparées les unes des autres par des ponts de tissu.

7. Dispositif (1) selon l'une quelconque des revendications 1 à 6, **caractérisé en ce que** le module de balayage (15) est conçu de façon à positionner des impulsions laser successives (L') de telle manière que leurs diamètres de foyer (P1, P2) se recouvrent partiellement.

8. Dispositif (1) selon la revendication 7, **caractérisé en ce que** leurs diamètres de foyer (P1, P2) se recouvrent au moins jusqu'à la moitié de leur diamètre.

9. Dispositif (1) selon l'une quelconque des revendications 1 à 8, **caractérisé en ce que** le module de balayage (15) est conçu de façon à déplacer le foyer (F) nettement plus rapidement qu'une vitesse de déplacement d'un oeil humain (2) lors d'un changement de direction du regard.

10. Dispositif (1) selon l'une quelconque des revendications 1 à 9, **caractérisé en ce que** le dispositif (1) comprend un module de surveillance de l'oeil (12) pour déterminer des mouvements de l'oeil, et **en ce que** le dispositif (1) est conçu de façon à commander le module de positionnement (16) en se basant sur les mouvements de l'oeil déterminés, en vue d'une compensation correspondante du positionnement.

11. Dispositif (1) selon l'une quelconque des revendications 1 à 10, **caractérisé en ce que** le motif de balayage (p) définit chaque fois une zone partielle de traitement (a) avec un forme pouvant être rectangulaire, ronde, elliptique, en étoile, en spirale ou en figure de Lissajou.

12. Dispositif (1) selon l'une quelconque des revendications 1 à 11, **caractérisé en ce que** le motif de balayage (p) définit une zone partielle de traitement (a), dont le diamètre est inférieur à l'épaisseur de la cornée (21).

13. Dispositif (1) selon l'une quelconque des revendications 1 à 11, **caractérisé en ce que** le module de balayage (15) comprend, pour dévier les impulsions laser, au moins un appareil parmi un galvanoscanneur, un scanner à miroir résonant, un modulateur optique acoustique, un scanner polygonal et un scanner micro-électromécanique.

14. Dispositif (1) selon l'une quelconque des revendications 1 à 13, **caractérisé en ce que** le module de positionnement (16) comprend des générateurs de mouvement pour le déplacement mécanique d'au moins des parties du projecteur de lumière (11).

15. Dispositif (1) selon l'une quelconque des revendications 1 à 14, **caractérisé en ce que** le module de positionnement (16) comprend un galvanoscanneur pour dévier les impulsions laser.

16. Dispositif (1) selon l'une quelconque des revendications 1 à 15, **caractérisé en ce que** le projecteur de lumière (11) présente une ouverture numérique de plus de 0,3.
